# EUROPEAN PATENT APPLICATION

(11) **EP 4 414 006 A1**
(43) Date of publication of application: **14.08.2024**
(21) Application number: 23155618.4
(22) Date of filing: 08.02.2023
(51) Int. Cl.: A61M 5/315, A61M 5/20, A61M 5/31, A61M 5/32, A61M 5/50

(54) **AN INJECTION DEVICE COMPRISING A CLOSURE CAP**

(71) Applicant: Ypsomed AG, 3401 Burgdorf (CH)
(72) Inventor: Bernhard, Mario, 3400 Burgdorf (CH); Bosshard, Simon Martin, 3324 Hindelbank (CH); Schrul, Christian, 3400 Burgdorf (CH); Zumstein, Dominik, 3014 Bern (CH)
(74) Representative: Meier Obertüfer, Jürg

(57) **Abstract**

An injection device for delivery of a fluid medicament including a housing (2) defining a longitudinal axis and enclosing an injection mechanism. An end cap (12) is fixated to the housing (2) thereby delimiting the injection mechanism in a proximal direction. A closure cap (17) closes the injection device (20) and is connected to the end cap (12) by a mechanical interface (12e,12f,17a) configured to be released by rotating the closure cap (17) relative to the housing (2) around the longitudinal axis. A locking plate (16c) is positioned between the closure cap (17) and the end cap (12), and the closure cap (17) is coupled to the locking plate (16c) by a first coupling (17b, 16d) and the locking plate (16c) is coupled to the end cap (12) by a second coupling (16f, 12d) thereby preventing rotation of the closure cap (17) with respect to the housing (2).

## Description

### TECHNICAL FIELD

The current invention relates to an injection device comprising a releasable connection for a closure cap closing the injection device

### BACKGROUND OF THE INVENTION

Injection or infusion devices such as injection pens, auto injectors, autopens, patch injectors or patch pumps use a drive mechanism for expelling medicament from the device. The drive mechanism comprises mechanical components for advancing, for example, a piston rod forwarding a bung in a reservoir or to drive a pumping mechanism. The injection or infusion devices may be disposable or reusable devices. For disposable injection or infusion devices, the mechanical components are disposed of after use and may be used for recycling specific components or the raw materials if the device is shredded or disassembled. Alternatively, the device is combusted or ends in a waste depository. The injection or infusion devices may require a dedicated waste treatment after use as they may comprise needles and may be chemically or biologically contaminated.

There is a need to include additional features to those mechanical devices for example by adding additional modules to the mechanical device. Examples for those additional modules may be an electronic module such as connectivity module with a transmitter and/or receiver to allow communication with other devices, a sensing module to allow for sensing patient parameters such as blood glucose values, a location module indicating geographical locations, a timer or calendar module, a communication or training module including an output module with a loudspeaker, or a sensing module e.g. to detect impact. Those additional electronic modules may be added as an add-on or supplementary device to the existing device. The add-on may be a separate add-on that the end user attaches to the injection or infusion device or the add-on is integrated into the mechanical device by the device manufacturer, a so called integrated add-on.

The separate add-on or the integrated add-on may itself be disposable or reusable. For a reusable add-on combined with a disposable injection or infusion device, the add-on must be removed from the disposable device for re-use of the add-on. For a disposable add-on it may be beneficial to separate the add-on from the mechanical components for separate waste treatment as most add-on devices include electronic components and batteries requiring a different treatment compared to the mechanical components.

There is a need to reduce waste and to facilitate re-use of components or materials or to recycle materials to reduce the amount of raw material or energy used throughout the lifecycle of the product. Thus there is a need to improve the injection device technologies including add-on features such that they are designed for recycling. The injection device may be disposed after use as being chemical/biological (contaminated) waste which must be treated differently from the electronic modules.

It is desired to encapsulate the modules comprising the add-on features in a housing part or closure cap that may be attached to the housing enclosing the mechanical components or to an end surface or end cap of the existing housing. The closure cap may protect the electronic components from contamination and mechanical damage. Additionally, the closure cap may ensure that the user cannot see or touch the components enclosed in the closure cap.

After use, the add-on may be removed from the injection or infusion device such that mechanical and electronic components can be separated for recycling purposes. This will require removal of the closure cap and/or the electronic components enclosed in the closure cap.

In US7008399 an injection pen is disclosed comprising a printed circuit board (PCB), a battery and a LCD located behind a window in the dose setting knob. The mechanical and electronic components of the pen are fully integrated and there are no precautions taken to separate the components.

In EP 4122512 A1 an autoinjector is disclosed with a module housing closing an electronic module that is releasable attached to a main housing comprising the injection mechanism. The module housing can be separated from the housing after injection.

WO2017032586 presents a monitoring unit for a medicament delivery device with an attachment mechanism comprising releasable locking elements for releasable attachment to the medicament delivery device.

It is an object of the present invention to improve the injection devices for recycling purposes by providing closure caps that can be securely mounted onto the injection device before use and released after use. These objectives are solved by the independent claim and specific variants are disclosed in the dependent claims.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "a locking plate" does not exclude the fact that there may be two locking plates that functionally or structurally fulfill the purpose of "a locking plate". The mere fact that certain elements or steps are recited in distinct claims shall not preclude the existence of further meaningful combinations of these elements or steps.

### GENERAL DESCRIPTION OF THE INVENTION

In an embodiment of the present invention an injection device or a medicament delivery device for delivery of a fluid medicament is presented comprising a housing defining a longitudinal axis and enclosing an injection mechanism. An end cap is fixated or configured to be fixated the housing thereby delimiting the injection mechanism in a proximal direction. A closure cap closes the injection device and is connected to the end cap or housing by a mechanical interface that is configured to be released by rotating the closure cap relative to the housing around the longitudinal axis. The injection device furthermore comprises a locking plate positioned between the closure cap and the end cap, and the closure cap is coupled to the locking plate by a first coupling, and the locking plate is coupled to the end cap by a second coupling thereby preventing rotation of the closure cap with respect to the housing, and thereby preventing release of the mechanical interface such that the closure cap cannot be removed from the injection device.

The injection device may be a pen type injection device, for example an auto-injector or an autopen. Alternatively, the injection device may be a patch injector configured to be attached to the skin of a patient. The housing may be a tubular housing for a pen type injection device.

The injection mechanism may be driven by a mechanical, electromechanical, electrical, hydraulic, or pneumatic power package. A mechanical power package may be based on a compression or torsional spring for driving the injection mechanism.

The end cap may be non-releasable fixated to the housing by a snap fit mechanism, or may be screwed, glued, laser-welded or ultrasonic-welded to the proximal end of the housing. Alternatively, the end cap may be releasable fixated to the housing, for example by a screw type connection. In case of a releasable connection, then this connection is not releasable when the closure cap is removed from the injection device. Alternatively, the end cap and the housing are made as one unitary component instead of two separate components. The end cap and housing may be injection molded as a monolithic part.

The closure cap may close the injection device and may cover and/or encapsulate the end cap for the injection mechanism. The end cap and the closure cap are preferably located at the proximal end of the injection device. A space may be available between the closure cap and the end cap and the space may be available for a separate mechanical, optical, electrical or display module. The closure cap and the module may be part of an add-on unit or a supplementary device for the injection device.

The closure cap may be made from a transparent material or from a non-transparent material. The closure cap may be flexible or rigid. The closure cap may be mechanically reinforced, for example made from a fibre reinforced plastic.

Optionally, the closure cap comprises shock absorbers to absorb forces that may act on the injection device during an impact, for example during a drop test. The shock absorber may be a layer of an elastomeric material applied to the outside surface of the closure cap or flexible arms, rims or wings may extend from the closure cap. The outside surface of the closure cap may have dedicated structures or a shape designed to fit into a dedicated tool for rotating the closure cap and applying torque to the closure cap.

The injection device or medicament delivery device may be a re-usable or disposable device and may be used without the add-on unit or with the add-on unit. The add-on unit may be re-used on a new device, for example if the previous device has been emptied. The add-on unit may therefore contribute to closing a recycling loop or reduce waste when used in combination with a disposable injection device.

The end cap closes the injection mechanism that is in the housing and the injection device with the closure cap and the add-on unit may be available as a single unit or the injection device may be available as a product without the add-on unit. The injection device may be manufactured including the add-on unit mounted or the add-on unit may be provided as a separate part. The injection device and the add-on unit may be provided as a kit of parts.

The closure cap is preferably attached to the end cap by the mechanical interface such that the closure cap and therewith the add-on unit is fixed to the injection device during use. Alternatively, the closure cap may be attached to the housing of the injection device by a mechanical interface. The closure cap and/or add-on unit may be removed from the injection device by release of the releasable mechanical interface. The closure cap is preferably rotated in a single rotation direction. Alternatively, combined movements, for example pull or push movements along the longitudinal axis combined with rotational movement around the longitudinal axis may release the mechanical interface.

A threshold torque may be required for releasing the closure cap from the injection device. If a torque is applied below the threshold value, the closure cap cannot be removed from the injection device which ensures that the closure cap and therewith the add-on unit is fixated to the injection device before or during use. After use, a torque exceeding the threshold may release the closure cap from the injection device. A dedicated tool may be used to apply torque to the closure cap. The mechanical interface prevents axial movement between the closure cap and the end cap or between the closure cap and the housing and preferably allows for rotational movement in one direction.

A locking plate may be positioned between the end cap and the closure cap. The locking plate may be part of the add-on unit. The locking plate may also be a locking disk or a driving plate. The first coupling may be a first coupling engagement or first connection between the closure cap and the locking plate. The first coupling may comprise first and second coupling members or first and second couplers that are configured to engage each other. The first coupling between closure cap and the locking plate preferably ensures that the closure cap is axially slidabe and rotationally coupled to the locking plate.

The second coupling may be a second coupling engagement or second connection between the locking plate and the end cap. The second coupling may comprise third and fourth coupling members or couplers on the locking plate and end cap, respectively. The third and fourth couplers or coupling members that are configured to engage each other. The second coupling between the locking plate and the end cap preferably ensures that the locking plate is axially and rotationally fixed to the end cap.

The first and second couplings prevent rotation of the closure cap relative to the housing or end cap by providing a threshold locking force via the locking plate. As the closure cap cannot be rotated, or cannot be rotated before the threshold torque is applied to the closure cap, this implies that the mechanical interface cannot be released and the closure cap or add-on unit cannot be removed from the injection device.

The mechanical strength of the first coupling or first coupling engagement or first connection preferably exceeds the mechanical strength of the second coupling or second coupling engagement or second connection. The mechanical strength of the engagement between the first and second coupling members preferably exceeds the mechanical strength of the engagement between the third and fourth coupling members. The locking plate or couplers on the locking plate preferably have a mechanical strength that exceeds the strength of the first coupling between the closure cap and the locking plate.

The second coupling is configured to be released, deformed or disrupted when the closure cap is rotated relative to the housing around the longitudinal axis, preferably when the torque applied to the closure cap exceeds the threshold value. The torque is transmitted from the closure cap to the locking plate via the first coupling and from the locking plate to the end cap via the second coupling. The second coupling may be irreversibly released or disrupted such that the locking plate and the closure cap are configured to allow a rotation with respect to the end cap or housing. During rotation of the closure cap, the mechanical interface between the closure cap and the end cap or housing is released such that the closure cap or the add-on unit may be removed from the injection device. The add-on unit or closure cap may be re-used on another injection device or may be re-cycled. The add-on unit may have electronic components that may easily be separated from a mechanical injection device.

The second coupling is irreversibly released such that the locking plate and therewith the electronic module may be used once only. Additionally, the end cap may be irreversibly deformed such that also the injection device may be used once only.

In an embodiment, the mechanical interface between the closure cap and the end cap or housing may be a bayonet fitting configured to be released by a relative rotation between the closure cap and the housing in one direction. The bayonet fitting preferably prevents axial movement between the closure cap and the end cap or housing and allows for rotational movement in one direction such that the closure cap may be removed from the injection device. The bayonet fitting may have a fifth coupling member on the closure cap, for example a protrusion that is configured to engage a sixth coupling member, for example a slot on the end cap or on the housing. Alternatively, the mechanical interface may be a screw type of engagement.

The first coupling may include a clearance fit between at least one protrusion on the closure cap engaging at least one aperture in the locking plate such that the closure cap is axially slidable but rotationally coupled to the locking plate (or the locking plate is axially slidable but rotationally coupled to the closure cap). The aperture in the locking plate is preferably oversized compared to the outer dimension of the at least one protrusion. The at least one protrusion may be tapered. Preferably two protrusions may be used. Alternative engagements for the first coupling may be a form fit or a positive locking fit or male/female type of connection or a magnetic engagement or an adhesive connection. The aperture on the locking plate may be a cut-out at an edge of the locking plate or a through hole within the locking plate.

The second coupling between the locking plate and the end cap may include an interference fit between at least one protrusion on the end cap engaging at least one aperture in, or cut-outs on the edge of the locking plate such that the locking plate is axially and rotationally secured to the end cap. An alternative type of engagement for the second coupling may be a form fit or a positive locking fit. The second coupling may be established using heat staking, laser welding or ultrasonic welding techniques. For example the at least one protrusion on the end cap may be subjected to heat staking while engaging a cut-out on the locking plate resulting in a non-releasable connection or coupling that may be disrupted provided sufficient torque is applied to the locking plate.

Preferably the at least one protrusion on the closure cap has a mechanical strength exceeding the strength of the at least one protrusion of the end cap such that the protrusion on the end cap is configured to be plastically and/or elastically deformed when the closure cap is rotated for releasing the mechanical interface. The at least one protrusion on the closure cap may have an outer diameter exceeding the outer diameter of the at least one protrusion on the end cap. A plurality of protrusions on the closure cap may exceed the mechanical strength of a plurality of protrusions on the end cap. The protrusions and/or the closure cap may be made from a material having a mechanical strength exceeding the mechanical strength of the material of the end cap. The closure cap may, for example, be made from a fiber reinforced material. The protrusions on the closure cap may be positioned radially outwards from the central axis of the device compared to the protrusions on the end cap,

In an embodiment, the closure cap or the add-on device for the injection device may enclose an electronic module capable of detecting and monitoring functions of the injection device or medicament delivery device. Such functions may include monitoring the start of an injection, monitoring of a device status during an injection, monitoring the end of injection, monitoring a holding time after an injection, or detecting the removal of the closure cap or add-on unit. Optionally, the electronic module comprises a transmitting device for transmitting data to an external device or the electronic module may comprise a receiver for receiving information from an external device. The electronic module may comprise a processor, an antenna or a memory unit. The electronic module may have a switch for activating the electronic module to save battery power. The switch may also be used to activate other monitoring functions provided by the electronic module.

The electronic module includes a printed circuit board (PCB) having a battery connected to the printed circuit board by a battery connection. The battery may provide electric power to the electronic module. The electronic circuitry of the electronic module may be powered when the add-on unit is attached to the injection device. The electronic module may be powered only during use of the add-on unit to extend the battery lifetime. The power may be switched off when the add-on unit is removed from the injection device such that the add-on unit has sufficient battery power when used on another injection device.

In a preferred embodiment the printed circuit board may be the locking plate and the locking plate may have a mechanical strength exceeding the strength of the at least one protrusion on the closure cap. Using the printed circuit board as a locking plate may ensure that no separate locking plate is required, thereby reducing the number of components needed for the add-on module.

The battery connection between the PCB and the battery may be configured to be disrupted when the closure cap is rotated for releasing the mechanical interface between the closure cap and the end cap. Once the battery connection is disrupted, the battery may be handled or disposed of separately from other components of the add-on unit.

The end cap may comprise a locking element preventing co-rotation of the battery with the printed circuit board when the closure cap is rotated for releasing the mechanical interface, such that the relative rotation between the printed circuit board and the battery interrupts or disrupts the battery connection and releases the battery from the printed circuit board. The battery connection may be interrupted meaning that the battery remains physically connected to the PCB but the electrical connection is interrupted. Alternatively, the battery connection may be disrupted such that the electrical connection is interrupted and the battery is physically separated from the PCB. The PCB, the closure cap and the battery may be available for recycling as separate parts and the integration of the battery removal from the PCB during removal of the closure cap may reduce the number of handling steps during recycling.

The printed circuit board preferably comprises a processor and an electronic circuit comprising an electronic switch, whereby the electronic switch may be configured to be actuated by the injection mechanism during an injection. The term "during an injection" may be defined as including the start and the end of an injection, whereby the injection ends when a predefined amount of medicament has been delivered to a patient.

In a preferred embodiment the electronic switch is configured to be actuated by rotation of the closure cap after the end of an injection, thereby monitoring the removal of the closure cap. The removal of the closure cap or removal of the add-on unit may be transmitted to an external device and/or may be accompanied by an audible signal (for example using a buzzer or loudspeaker) or a visual signal (for example using a LED or a display) generated by the add-on unit.

Preferably, the electronic switch is actuated before interrupting the battery connection. A preferred sequence may be to start an injection and at the start or during medicament delivery the electronic switch of the electronic module may be actuated. The electronic module may monitor the start of the injection and optionally transmit a signal to an external device. The electronic module may comprise a communication module or a display module which may indicate the start of the injection to the user of the device. At the end of the injection, the injection device may activate the switch for a second time, thereby indicating to the user or the external device that the injection has been completed. As a final step, a torque may be applied to the closure cap exceeding the threshold torque for disrupting the second coupling between the locking plate and the end cap such that the closure cap may be rotated allowing to start the release of the mechanical interface between the closure cap and the end cap or housing. During rotation of the closure cap the electronic switch may be actuated a third time, thereby indicating to the user or the external device that the add-on unit has been removed. After the third actuation, the mechanical interface between the closure cap and the end cap or housing is released and the add-on unit may be removed from the injection device. Optionally, the battery connection is disrupted before removing the closure cap from the injection device.

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

### LEGENDS TO THE FIGURES

- Figure 1:: Exploded view of an autoinjector,
- Figure 2a:: Longitudinal sectional view of the autoinjector,
- Figure 2b:: Longitudinal sectional view of the autoinjector, in a view perpendicular to Figure 2a,
- Figure 3a:: The autoinjector shown with an exploded view of the closure cap, the locking plate and the end cap,
- Figure 3b:: Longitudinal sectional view of the autoinjector ready to use,
- Figure 3c: Longitudinal sectional view of the autoinjector after use,
- Figure 4:: End cap for the auto injector,
- Figure 5a:: Cross section of closure cap, end cap of the autoinjector before use showing the mechanical interface between the closure cap and the end cap,
- Figure 5b:: Cross section of closure cap, end cap of the autoinjector after use, closure cap twisted,
- Figure 6a:: Cross section of closure cap, end cap of the autoinjector before use showing the engagement between the closure cap and the locking plate and between the locking plate and the end cap,
- Figure 6b:: Cross section of closure cap, end cap of the autoinjector after use with twisted closure cap,
- Figure 7a:: Detail of the sensor/switch on the PCB before rotation of the closure cap,
- Figure 7b:: Detail of the sensor/switch on the PCB after rotation and removal of the closure cap,
- Figure 8a:: Assembly of end cap with integrated battery separator and closure cap before rotation of the closure cap,
- Figure 8b:: Assembly of end cap with integrated battery separator and closure cap after rotation of the closure cap.

### DETAILED DESCRIPTION OF THE FIGURES

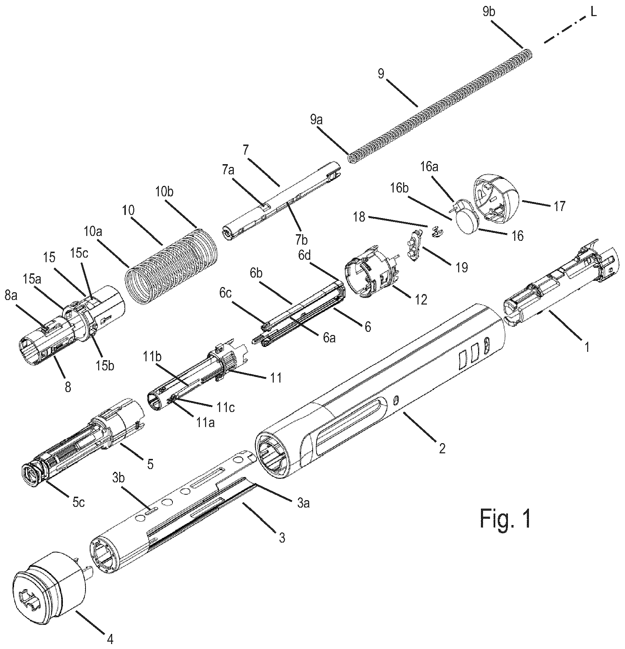

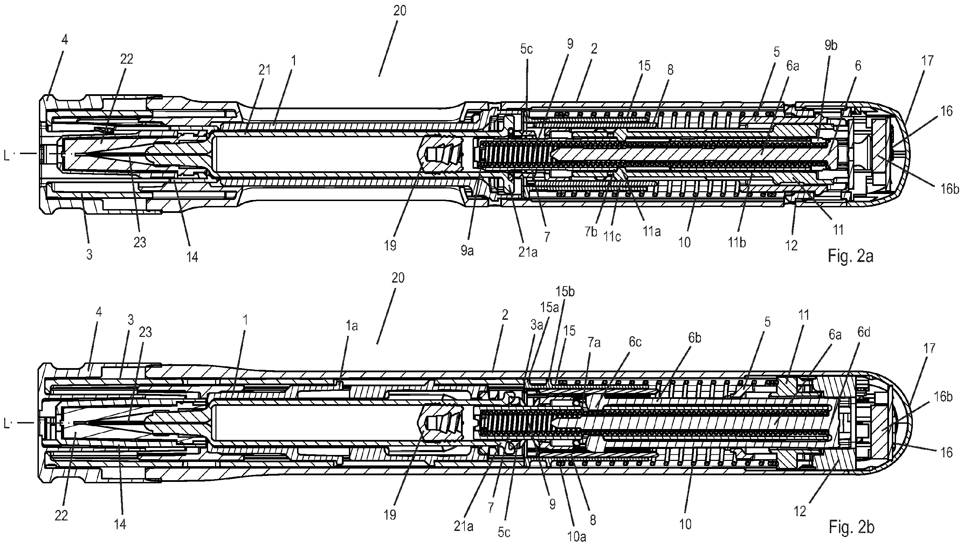

Figure 1 presents an exploded view of an autoinjector. An elongated sleeve shaped housing 2 can be gripped by the user during an injection and the housing defines a longitudinal axis L. An injection mechanism that is configured to be driven by a coiled compression spring 9 is enclosed in the housing 2. To illustrate the function of the components, Figures 2a and 2b depict longitudinal sectional views of the autoinjector 20. A prefilled syringe 21 is received in a syringe holder 1 and the syringe holder 1 is axially and rotationally secured to the housing 2 with a snap-fit connection. The autoinjector 20 as delivered to the patient is closed at the distal end by a pen cap 4 that must be removed before using the autoinjector. A needle shield 22 of the prefilled syringe 21 is coupled to the cap 4 by a needle shield remover 14 such that removal of the pen cap 4 also removes the needle shield 22 thereby exposing hollow needle 23. A needle cover sleeve 3 is axially guided by, and can be moved relative to the housing 2 along the longitudinal axis L a distance corresponding to an actuation hub. The needle cover sleeve 3 can slide in the proximal direction for actuating the injection mechanism to expel medicament from the autoinjector 20. A mechanic holder 5 is snap-fitted into and rotationally and axially secured to the housing 2. The mechanic holder 5 comprises an elastic element 5c (holding spring) at the distal end which abuts the proximal end 21a of the prefilled syringe 21 and biases the prefilled syringe distally into the syringe holder 1.

The injection mechanism comprises a compression spring acting as an injection spring 9. The injection spring 9 in its initial state is almost completely enclosed by a piston rod 7 and the distal end 9a of the injection spring 9 biases the piston rod 7 into the distal direction. The proximal end 9b of the injection spring abuts a holding element 6 and the holding element 6 comprises two arms 6b extending from a proximal end 6d in the distal direction and one central pin 6a for guiding the injection spring 9. Each arm 6b comprises a protrusion 6c directed towards the axis L and both protrusions engage recesses 7a on the outer surface of the piston rod 7. In the initial state of the autoinjector, a switching module 8, 15 comprising switching sleeve 15 and a locking sleeve 8 prevent a deflection of the arms 6b as the arms 6b are confined within the locking sleeve 8 and therewith prevent a relative movement between the piston rod 7 and the holding element 6 thus keeping the injection spring 9 in a compressed state. The piston rod 7 is prevented from movement into the distal direction. At least the distal end 15a of the switching sleeve 15 is engaged or abuts with a proximal end 3a of the needle cover sleeve 3. The switching sleeve 15 is biased by a needle cover sleeve spring 10 into the distal direction. The distal end 10a of the needle cover sleeve spring 10 abuts a rim 15b on the switching sleeve 15 and the proximal end 10b of the needle cover sleeve spring 10 abuts a signaling element 11 and the signaling element abuts an end cap 12 that is axially and rotationally secured to the housing 2. In the initial state of the autoinjector 20, the force of the needle cover sleeve spring 10 in the proximal direction is guided to the end cap 12 of the housing 2 via the signaling element 11. In the distal direction, the needle cover sleeve spring 10 biases the needle cover sleeve 3 into the distal direction via the switching sleeve 15. For starting the injection, the needle cover sleeve 3 is pushed against the skin of the patient and moved in the proximal direction with respect to the housing 2 thereby moving the switching module 15, 8 in the proximal direction and tensioning the needle cover sleeve spring 10. In the most proximal position of the switching module 15, 8, the locking sleeve 8 may be axially engaged with the mechanic holder 5 (or alternatively to the signaling element 11 as will be discussed below) whereas the switching sleeve 15, axially guided and splined onto the locking sleeve 8, is kept by the needle cover sleeve 3 in the proximal position against the bias of the spring force provided by the needle cover sleeve spring 10.

The proximal movement of the switching module 8, 15 releases the engagement between the protrusions 6c on the holding element 6 and the recesses 7a in the piston rod 7. The spring force is released and the piston rod 7 moves into the distal direction whereas the holding element 6 can move an initial hub in the proximal direction until the proximal end 6d of the holding element 6 abuts the end cap 12 of the housing 2. Optionally, an acoustic or tactile signal is generated upon abutment indicating the start of the injection. The piston rod 7 abuts a bung 19 in the prefilled syringe which is moved distally towards the outlet thereby expelling medicament through the needle 23.

The needle cover sleeve spring 10 is a metal coil spring acting as a compression spring and the proximal end 10b abuts the signaling element 11 which abuts the end cap 12 of the housing 2 before injection. The signaling element 11 comprises two longitudinally extending arms 11b each having a protrusion 11a oriented towards the longitudinal axis L. The protrusions 11a engage another recesses 7b in the piston rod 7 and the sleeves of the switching module 8, 15 ensure that the signaling element 11 is axially coupled to the piston rod 7 before and during the initial stage of the injection. The arms 11b with protrusions 11a are also confined within the locking sleeve 8 thereby preventing release of the protrusions from the recesses. During the initial stage of the injection, the piston rod 7 is advanced distally and thereby also drives the signaling element 11 in the distal direction away from the end cap 12 through the locking sleeve 8. The signaling element 11 is moved in the distal direction by a distance or tensioning hub until the protrusions 11a are released from the recesses 7b in the piston rod 7 as the arms 11b are not confined by the locking sleeve 8 anymore and can flex radially outward. The arms 11b have another protrusion 11c that is directed in the radial outward direction and those outward protrusions 11c engage the switching module 8, 15 keeping the tensioning hub of the signaling element 11. The arms 11c preferably engage the locking sleeve 8 thereby keeping the locking sleeve 8 in the proximal position (alternatively the locking sleeve engages the mechanic holder 5 as discussed above). The arms 11b can slide along the outer surface of the piston rod 7 and at the end of the injection, the piston rod has advanced such that the arms 11b of the signaling element 11 can flex radially inward again and the signaling element 11 is released from the switching module 8, 15. The signaling element 11 is biased proximally by the needle cover sleeve spring 10 and is accelerated and moved back towards the end cap 12 thereby producing an audible and/or tactile end-of-injection click.

After the medicament has been delivered, the autoinjector 20 is removed from the skin and the switching sleeve 15 together with the needle cover sleeve 3 are forwarded by the needle cover sleeve spring 10 that is at least partially decompressed. The switching sleeve 15 moves in the distal direction and is sliding along the locking sleeve 8 which has been locked before injection to the mechanic holder 5. The switching sleeve 15 may be locked in the most distal position by a flexible arm 8a of the locking sleeve 8 engaging a cut out 15c of the switching sleeve 15 thereby preventing proximal movement of the switching sleeve 15 after injection. The needle cover sleeve 3 may be locked in the most distal position by arms 1a of the syringe holder 1 that radially extend into recesses 3b of the needle cover sleeve 3 thereby preventing proximal movement of the needle cover sleeve 3 after injection.

The end cap 12 is axially and rotationally fixed to the housing 2. The end cap 12 may be snap fitted, adhesively connected or welded to the housing 2. The end cap 12 can be the closure cap for the autoinjector delimiting the mechanical parts of the device in the proximal direction if there are no further modules added to the autoinjector. The end cap may provide protrusions, ribs or 3D structures acting as shock absorbers. Preferably, the end cap 12 provides a platform or adapter for adding an additional module to the autoinjector. Preferably this is an electronic module 16 and the module is closed by a closure cap 17 engaging the end cap 12 and/or housing 2 of the autoinjector. The closure cap 17 may provide protrusions, ribs or 3 D structures on the outer surface acting as shock absorbers. Preferably, the module is a releasable module that can be released and separated from the autoinjector after injection. The electronic module 16 may comprise a sensor or switch 16a and a battery 16b and a printed circuit board 16c. The printed circuit board 16c may comprise a microprocessor, a transmitter/receiver unit, an antenna and/or a light source such as a LED. The light source may be used to signal the status of the device to the user, for example "ready to use" or "injection completed". The sensor 16a may be part of the PCB 16c as well and is configured to detect if the signaling element 11 is in the most proximal position. The sensor 16a may be a switch located on the PCB 16c detecting whether the signaling element 11 is in its most proximal position before injection and/or the most proximal position after injection and/or the absence of contact to the sensor during an injection. The signaling element 11 may have a protrusion extending into the proximal direction and optionally guided through a passage in the end cap 12 for contacting the sensor 16a. Alternatively, the signaling element 11 indirectly contacts the sensor 16a, for example via a pivoting element, a rotating element or an element that moves along the pen axis 18 that is part of or coupled to the end cap 12. The microprocessor detects the signals from the sensor 16a and is capable to transmit the data (for example time, duration, failure) of an injection to an external device using a communication module comprising, for example a transmitter and/or receiver.

The signaling element 11 or an extension of the signaling element 11 may therefore contact the sensor 16a directly or indirectly before an injection as the signaling element is in the most proximal position. During the injection the signaling element is driven by the piston rod 7 in the distal direction and the signaling element 11 or an extension therefrom may not contact the sensor 16b. At the end of the injection, the signaling element 11 moves in the proximal direction and may contact the sensor 16b again. Each event may be recorded and may be transmitted to an external device or may trigger a visual or acoustic signal by the electronic module.

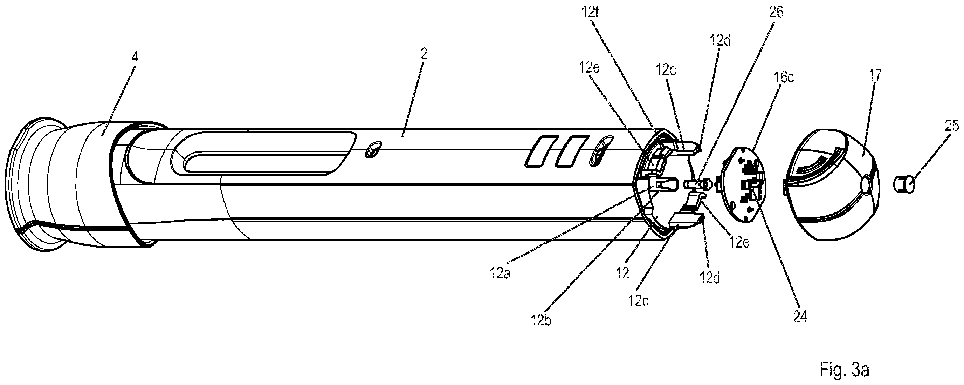

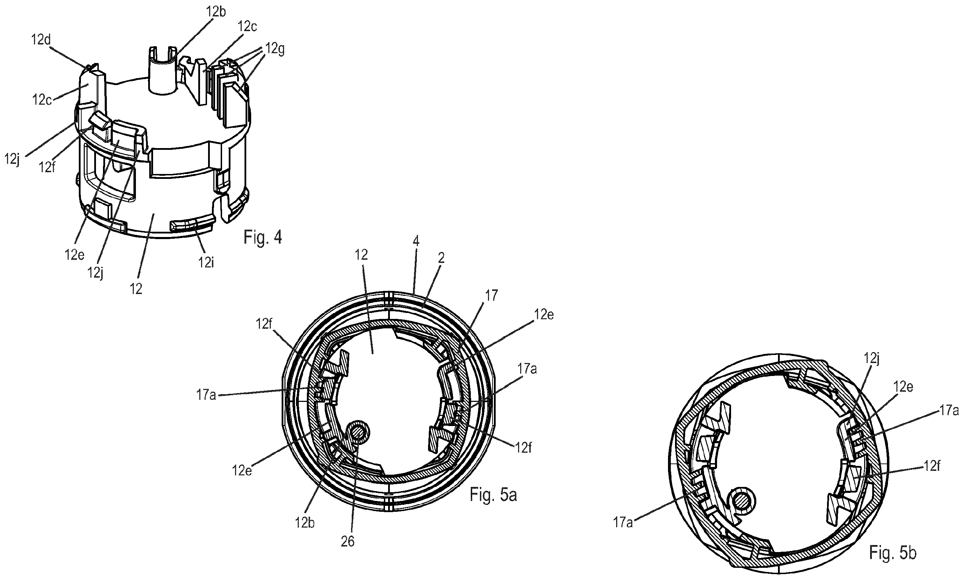

Figure 3a presents an exploded view of an autoinjector before use. The proximal end of the housing 2 is closed by the end cap 12 and the autoinjector is configured to be closed by the closure cap 17. A locking plate, in this example the PCB 16c, may be positioned between the closure cap 17 and the end cap 12. Alternatively, a separate locking disc or plate is used, for example a metal or plastic plate. Using the PCB also as a locking plate may reduce the number of parts required for the cap assembly. The end cap 12 comprises a passage 12a for a detection pin 26. The distal end of the detection pin 26 is configured to engage the drive mechanism of the autoinjector, for example the signaling element 11 or the holding element 6. The proximal end of the detection pin 26 is configured to engage the switch or sensor 16a on the PCB 16c. A guide sleeve 12b extends from the end cap 12 and the guide sleeve surrounds the passage 12a and supports the detection pin 26 such that the detection pin can move within the guide sleeve along the longitudinal axis L of the autoinjector. Protrusions 12c extend from the end cap 12 in the proximal direction ending in proximal extensions 12d. The extensions 12d are configured to engage the PCB 16c. The end cap furthermore comprises a bayonet slot 12e and a bayonet lock 12f (Figure 4) which are configured to engage a protrusion 17a on the inside surface of the closure cap 17 (Figure 5a) to form a bayonet fitting 12e,12f,17a. The PCB 16c in Figure 3a comprises an LED light source 24 and the closure cap 17 has an opening with a transparent section or plug 25 for visual signaling.

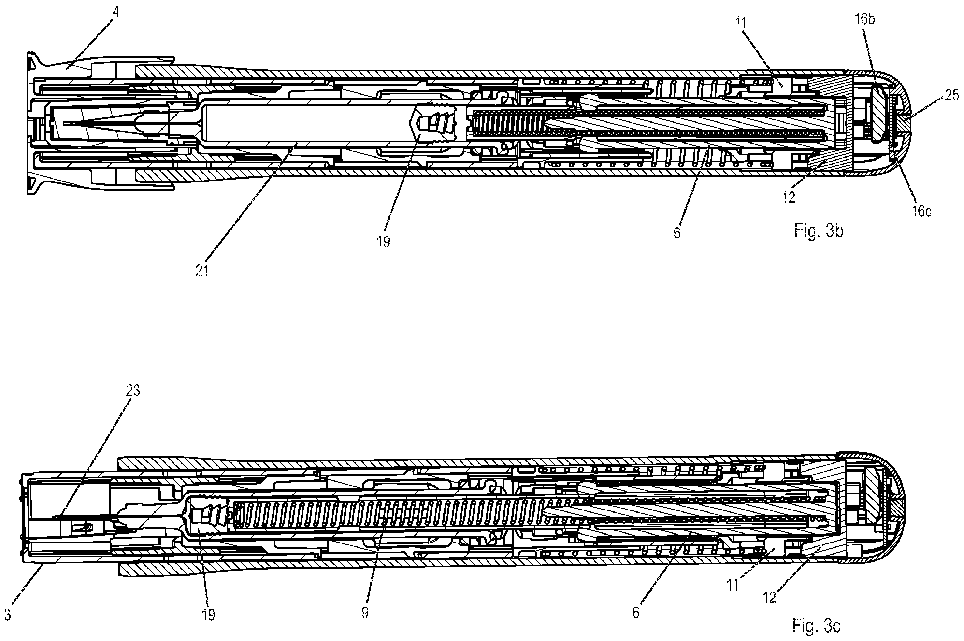

A longitudinal sectional view of the assembled auto injector of Figure 3a is shown in Figure 3b. The pen cap 4 has not been removed and the bung 19 in the prefilled syringe 21 is in the initial position before medicament delivery. The closure cap is mounted and the bayonet fitting 12e, 12f, 17a provides a mechanical interface between the closure cap 17 and the end cap 12 fixating the closure cap to the proximal end of the autoinjector. The signaling element 11 and the holding element 6 are in their respective initial positions before use. The signaling element 11 abuts the end cap 12 and therewith ensures that the detection pin 26 is in the most proximal position and the holding pin acts on the sensor 16a. The electric circuitry on the PCB may be closed or open when the detection pin acts on the switch. Additionally, the function of the switch may change from closing or opening a circuitry, followed by signaling the start or end of the injection, followed by indicating removal of the closure cap. Additionally, the electric circuitry may be in a sleeping mode before use such that, although being closed, a minimum of electric energy is consumed. A gap exists between the holding element 6 and the end cap 12 (Figure 3b) which is closed at the start of the injection as the holding element moves in the proximal direction. The holding element 6 and/or the detection pin 26 may be configured to be moved by the signaling element 11 only, by the holding element 6 only or by both the signaling element 11 and the holding element 6.

Another longitudinal section of the assembled auto injector of Figure 3a is shown in Figure 3c. The pen cap 4 has been removed for injecting the medicament and the bung 19 in the prefilled syringe is in the final position after medicament delivery. The needle cover sleeve 3 is in the extended (distal) position again and the injection spring 9 is expanded. The holding element 6 has been moved by the compression spring 9 at the start of the injection in the proximal direction until the holding element 6 abuts the end cap 12. The signaling element 11 has been moved first in the distal direction during the injection and at the end of the injection been moved by the needle cover sleeve spring 10 into its most proximal direction thereby abutting the end cap 12 and providing the end of injection click. The closure cap 17 in Figure 3c is still mounted onto the injection device by the bayonet fitting.

A detailed view of the end cap 12 is depicted in Figure 4. Snap fit connectors 12i are located at the distal end of the end cap which are configured to engage corresponding connectors on the housing 2. Two bayonet slots 12e and bayonet locks 12f may engage two protrusions 17a on the closure cap 17. Two guide 12j oriented parallel to the longitudinal axis limits the degree of axial rotation of the protrusion 17a in the guide slot 12e in both rotational directions. The guide slot 12j may also facilitate insertion of the protrusion 17a on the closure cap into the bayonet fitting. Optionally, guide features separate from the bayonet fitting may facilitate the axial orientation and rotation of the closure cap 17 with respect to the end cap 12. The end cap may have additional protrusions 12g and its function will be described below. There may be embodiments with the additional protrusions 12g and without the additional protrusions 12g.

A cross section of the closure cap 17 before use of the device is shown in Figure 5a. The pen cap 4 is mounted onto the injection device. The protrusions 17a engage the bayonet lock 12f and the bayonet fitting is in the locked position. The closure cap can be rotated in one direction only if a threshold torque has been applied to the closure cap as will be explained below. The detection pin 26 is guided in the guide sleeve 12b such that the detection pin is aligned with the sensor 16a on the PCB. The closure cap has been rotated or twisted in Figure 5b until the protrusion 17a on the closure cap abuts the axial guide 12j on the end cap 12. The protrusions 17a are released from the bayonet locking protrusion 12f and the protrusions 17a enter the bayonet slot 12e such that the closure cap 17 may be removed from the injection device. The PCB 16a and/or the closure cap may be configured such that the PCB can be removed together with the closure cap or the two parts may be separately removed.

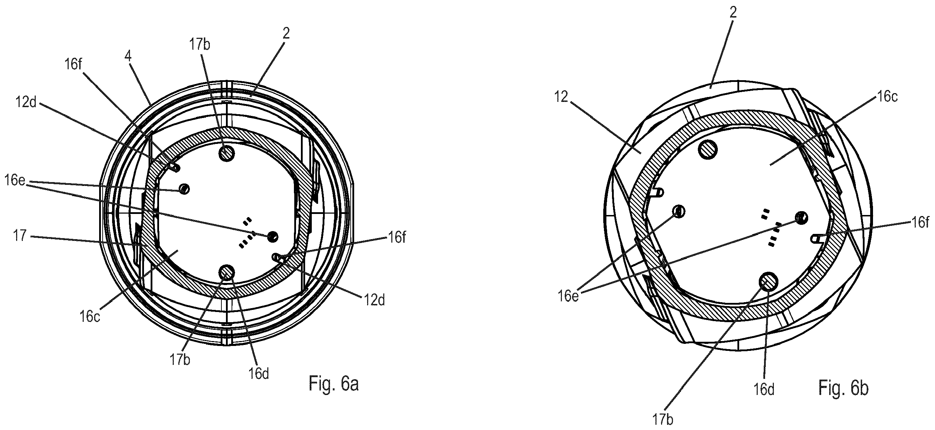

A cross section of the closure cap 17 before use of the device is shown in Figure 6a including the PCB or locking plate 16c located between the closure cap 17 and the end cap 12. The pen cap 4 is still mounted to the injection device and the closure cap 17 is in the original (non-twisted) position. The PCB 16c includes cut outs 16f engaging the extensions 12d on the end cap 12. The extensions 12d are preferably heat staked to provide an axial and rotational coupling between the PCB 16c and the end cap 12. The PCB 16c is therewith fixated to the end cap 12. The closure cap 17 comprises protrusions 17b that extend along the longitudinal axis L in the distal direction. The protrusions 17b engage passages 16d in the PCB 16c. The passages in the PCB may be oversized compared to the outer dimensions of the protrusions 17b or there may be a press-fit. The engagement 17b, 16d ensures that rotational torque applied to the closure cap 17 is transmitted to the PCB 16c. The PCB 16c acts as a locking plate preventing the rotation of the closure cap 17 with respect to the housing 2 or the end cap 12. The engagement 16f,12d between the PCB 16c and the end cap 12 is configured to be released or disrupted when a threshold torque is applied to the PCB 16c via the engagement 17b, 16d between the closure cap 17 and the PCB 16c. For example, the extensions 12d on the posts 12c may be plastically deformed when a threshold torque is applied thereby allowing rotation of the PCB 16c with respect to the end cap 12 and also allowing rotation of the closure cap 17. The engagement between the posts 12c and the PCB 16c may also be an adhesive connection, a welded connection (for example ultrasonic welding). In the example shown in Figure 6a, the engagement between the protrusions 17b on the closure cap and the passages 16d have a mechanical strength exceeding the strength of the engagement 12d, 16f between the end cap 12 and the PCB 16c. The outer dimensions of the protrusions 17b are greater compared to the outer dimensions of the extensions 12d in the embodiment presented in Figure 6a. Once the threshold torque is applied to the closure cap, the cap may be rotated together with the PCB as is shown in Figure 6b. Rotation of the closure cap 17 releases the bayonet fitting between the closure cap 17 and the end cap 12 (Figure 5b) such that the closure cap and/or the PCB can be removed from the injection device. The PCB 16c furthermore comprises two battery connectors 16e connecting the battery or a battery holder to the PCB. The battery connectors 16e and therewith the battery 16b are configured to co-rotate with the PCB in this embodiment.

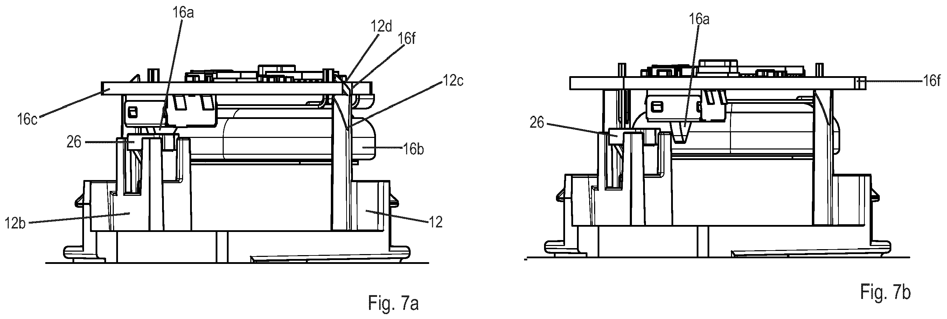

The detection pin 26 preferably contacts the sensor or switch 16a before use of the injection device and the PCB 16c is connected to the end cap 12 via the engagement between the (heat staked) extensions 12d engaging the cut outs 16f. The situation before use is shown in Figure 7a (closure cap not shown). After use, the PCB 16c may be rotated together with the closure cap and the coupling 12d, 16f between the PCB 16c and the end cap 12 is disrupted. The closure cap may be removed by releasing the bayonet fitting. The rotation of the PCB 16c is visualized in Figure 7b and the sensor 16a has been rotated such that the detection pin 26 does not contact the sensor 16a anymore. The sensor 16a may thus indicate the rotation of the closure cap after use.

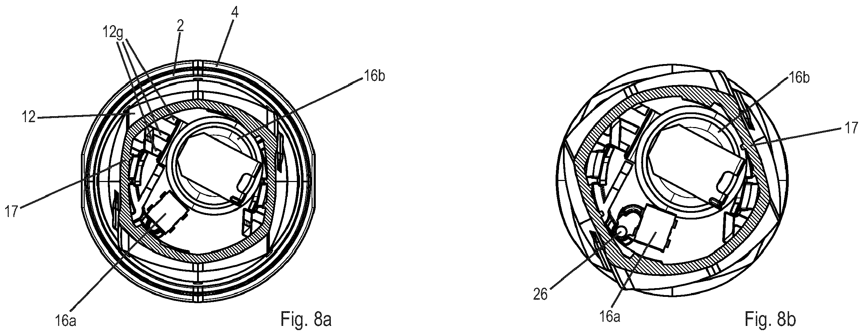

The rotation of the PCB 16c during closure cap removal may also be used to separate the battery 16b from the PCB 16c. The battery 16b is connected to the PCB via battery connectors 16e (Figures 5a and 5b) and the battery is configured to follow the rotation of the PCB 16c. In the embodiment presented in Figures 8a and 8b the end cap 12, which is fixated at the proximal end of the housing, has been provided with protrusions 12g that protrude into the proximal direction and which are intended to prevent rotation of the battery 16b. When the PCB is rotated by the closure cap, then the battery connectors 16e will co-rotate with the PCB (Figures 6a and 6b) whereas the battery itself will abut the protrusions 12g which cannot rotate as the end cap 12 is rotationally fixed to the housing. As a consequence, the battery connectors 16e will be disrupted and the battery is separated from the PCB during rotation of the closure cap such that the battery is immediately available for recycling.

A sequence for actuating the sensor or switch 16a is presented in the following. Before use of the injection device, the pen cap 4 is mounted and the detection pin 26 may be held against the switch 16a. The electronic circuitry of the electronic module 16 may be awake or may be in a sleeping mode. A sleeping mode is defined in that the electronic circuitry is temporarily active (pulsed) at a low frequency (for example only once in a minute) to reduce battery consumption. Alternatively, the electronic circuitry is always powered but certain parts of the module (for example the transmitter) are not powered by the battery. At the start of the injection, the signaling element 11 is moved into the distal direction and the detection pin 26 is moved by the signaling element 11 or by gravitational forces, or by a resilient element in the switch 16a into the distal direction as well. The sensor or switch 16a is actuated and this may wake up the electronic circuitry or may trigger an acoustic or visual signal from the electronic module indicating the start of the injection to the user. The electronic module may send also a signal to an external device using the transmitter indicating the start of the injection. At the start of the injection also the holding element 6 is moved into the proximal direction and the holding element may also act on the detection pin 26. At the end of the injection, the signaling element 11 is moved back towards the end cap 12 and may provide an audible end of injection click. The signaling element 11 may act again on the switch 16a via the detection pin 26 and this may trigger an acoustic or visual signal from the electronic module 16 and/or trigger a signal at an external device via the transmitter. Additionally, the electronic module 16 may start a holding time and at the end of the holding time trigger another optical and/or acoustic signal indicating to the user when the injection device may be removed from the skin. When the closure cap is removed from the device, the electronic switch 16a may be actuated once again (Figures 7a, 7b) and this may trigger a further acoustic and/or visual signal from the electronic module 16 indicating that the device is ready for disposal. Additionally, a signal may be sent to an external device. The signal sent to the external device may be used to trigger further actions such as sending a message to a health care professional who may decide on sending a replacement device to the patient. The replacement device is automatically sent to the user. A retrieval-kit may be sent to the user as well allowing the user to send the empty device and/or the removed add-on unit to a dedicated recycling company.

**PART ANNOTATION**

| | | | |
|---|---|---|---|
| 1 | Syringe holder | 12e | Bayonet slot |
| 1a | Arms | 12f | Bayonet lock |
| 2 | Housing | 12g | Protrusions (battery retainers) |
| 3 | Needle cover sleeve | 12i | Snap fit connector |
| 3a | Proximal end | 12j | Axial guide |
| 3b | Recess | 14 | Needle shield remover |
| 4 | Pen cap | 15 | Switching sleeve |
| 5 | Mechanic holder | 15a | Distal end |
| 5c | Holding spring | 15b | Rim |
| 6 | Holding element | 15c | Cut out |
| 6a | Central pin, guide pin | 16 | Electronic module |
| 6b | Arms | 16a | Sensor/switch |
| 6c | Protrusion | 16b | Battery |
| 6d | Proximal end | 16c | PCB, locking plate |
| 7 | Piston rod | 16d | Passage, second coupling member |
| 7a | Recess | | |
| 7b | Recess | 16e | Battery connector |
| 8 | Locking sleeve | 16f | Cut out, third coupling member |
| 8a | Flexible arm | | |
| 9 | Injection spring | 17 | Closure cap |
| 9a | Distal end | 17a | Protrusion for bayonet lock, fifth coupling member |
| 9b | Proximal end | | |
| 10 | Needle cover sleeve spring | 17b | Protrusion, first coupling member |
| 10a | Distal end | | |
| 10b | Proximal end | 18 | Pivoting element |
| 11 | Signaling element | 19 | Bung |
| 11a | Protrusion | 20 | Auto injector |
| 11b | Arms | 21 | Prefilled syringe |
| 11c | Protrusion | 21a | Proximal end |
| 12 | End cap | 22 | Needle shield |
| 12a | Passage | 23 | Hollow needle |
| 12b | Guide sleeve | 24 | LED |
| 12c | Protrusion | 25 | Transparent window |
| 12d | Extension, fourth coupling member | 26 | Detection pin |
| | | L | Longitudinal axis |
| 8,15 | Switching module | 12e,12f,17a | Bayonet fitting |
| 12e,12f | Sixth coupling member | | |
| 17b, 16d | First coupling | | |
| 16f, 12d | Second coupling | | |

## Claims

1. An injection device for delivery of a fluid medicament comprising
a housing (2) defining a longitudinal axis and enclosing an injection mechanism,
an end cap (12) fixated to the housing (2) thereby delimiting the injection mechanism in a proximal direction,
a closure cap (17) closing the injection device (20) and being connected to the end cap (12) by a mechanical interface (12e,12f,17a) configured to be released by rotating the closure cap (17) relative to the housing (2) around the longitudinal axis,
**characterized by** a locking plate (16c) positioned between the closure cap (17) and the end cap (12), wherein the closure cap (17) is coupled to the locking plate (16c) by a first coupling (17b, 16d) and wherein the locking plate (16c) is coupled to the end cap (12) by a second coupling (16f, 12d) thereby preventing rotation of the closure cap (17) with respect to the housing (2).

2. The injection device according to claim 1 wherein the mechanical strength of the first coupling (17b, 16d) exceeds the mechanical strength of the second coupling (16f, 12d).

3. The injection device according to claims 1 or 2 wherein the second coupling (16f, 12d) is configured to be released or disrupted when the closure cap (17) is rotated relative to the housing (2) around the longitudinal axis with a torque exceeding a threshold value.

4. The injection device according to any of the previous claims wherein the mechanical interface (12e, 12f, 17a) includes a bayonet fitting configured to be released by a relative rotation between the closure cap (17) and the housing (2) in one direction.

5. The injection device according to any of the previous claims wherein the first coupling (17b, 16d) comprises a clearance fit between at least one protrusion (17b) on the closure cap (17) engaging at least one aperture (16d) in the locking plate (16c) such that the locking plate (16c) is axially slidable but rotationally coupled to the closure cap (17).

6. The injection device according to claim 5 wherein the second coupling (16f, 12d) comprises an interference fit between at least one protrusion (12d) on the end cap (12) engaging at least one aperture in, or cut out on the edge of the locking plate (16c) such that the locking plate is axially and rotationally secured to the end cap (12).

7. The injection device according to claim 6 wherein the at least one protrusion on the closure cap (17b) has a mechanical strength exceeding the strength of the at least one protrusion (12d) of the end cap such that the protrusion (12d) on the end cap (12) is configured to be plastically and/or elastically deformed when the closure cap (17) is rotated for releasing the mechanical interface (12e, 12f, 17a).

8. The injection device according to any of the previous claims wherein the closure cap (12) encloses an electronic module (16) capable of detecting and monitoring functions of the injection device (20).

9. The injection device according to claim 8 wherein the electronic module (16) comprises a printed circuit board (16c) and a battery (16b) connected to the printed circuit board by a battery connection (16e).

10. The injection device according to claim 9 wherein the printed circuit board (16c) is the locking plate having a mechanical strength exceeding the strength of the at least one protrusion on the closure cap (17b).

11. The injection device according to claim 10 wherein the battery connection (16e) is configured to be disrupted when the closure cap (17) is rotated for releasing the mechanical interface (12e, 12f, 17a) between the closure cap (17) and the end cap (12).

12. The injection device according to claim 11 wherein the end cap (12) comprises a locking element (12g) preventing co-rotation of the battery (16b) with the printed circuit board (16c) when the closure cap (17) is rotated for releasing the mechanical interface (12e, 12f, 17a) such that the relative rotation between the printed circuit board (16c) and the battery (16b) interrupts or disrupts the battery connection (16e) and releases the battery (16b) from the printed circuit board (16c).

13. The injection device according to any of claims 9 to 12 wherein the printed circuit board (16c) comprises a processor and an electronic circuit comprising an electronic switch (16a) and whereby the electronic switch (16a) is configured to be actuated by the injection mechanism during an injection.

14. The injection device according to claim 13 wherein the electronic switch (16a) is configured to be actuated by rotation of the closure cap (17) after an injection.

15. The injection device according to claim 14 wherein the electronic switch (16a) is actuated before interrupting the battery connection (16e).
